# EUROPEAN PATENT APPLICATION

(11) **EP 2 399 514 A1**
(43) Date of publication of application: **28.12.2011**
(21) Application number: 11170721.2
(22) Date of filing: 21.06.2011
(51) Int. Cl.: A61B 5/11, A61B 5/00, G06F 19/00

(54) **Method for producing information for supporting home care service**

(30) Priority: 22.06.2010 FI 20105721
(71) Applicant: Vivago Oy, 00210 Helsinki (FI)
(72) Inventor: Nikkola, Ari, 02130 Espoo (FI); Myllymäki, Marko, 01600 Vantaa (FI)
(74) Representative: LEITZINGER OY

(57) **Abstract**

A method for producing information in support of homecare service for a person. The method comprises measuring the activity level with one or more motion and/or acceleration sensors and processing the measurement data for obtaining motion intensity readings from a measuring signal. In addition, it is interpreted that the person is outside when the measuring signal is no longer detected or when the signal strength is below a given level. The measuring result is compared with one or more threshold values and, as a result of the comparison, conclusions are made regarding the physical state of a person. The conclusions include sleeping times, outside visiting times, and circadian rhythm which is calculated by comparing nocturnal activity and diurnal activity. The results of conclusion are displayed on a remote monitoring terminal as a detailed report, e.g. in tabular format.

## Description

The invention relates to a method for producing information in support of homecare service for a person, said method comprising
- measuring the activity level with one or more motion and/or acceleration sensors
- processing the measurement data for obtaining motion intensity readings from a measuring signal
- interpreting that the person is outside when the measuring signal is no longer detected or when the signal strength is below a given level.

This type of method is known e.g. from the Applicant's patent publication EP 0724402 B1.

A shortcoming in this and also other prior known methods is that the measurement data is not refined far enough and into a suitable mode for readily using the information in support and enhancement of homecare services.

It is an object of the invention to eliminate this shortcoming and to provide a method of the foregoing type, wherein the motion activity data is communicated and refined, such that a remote monitoring terminal enables almost a real-time surveillance and tracking of the status and status progressing trends of persons.

This object is achieved by means of the method features presented in the appended claim 1. Preferred embodiments of the invention are presented in the dependent claims.

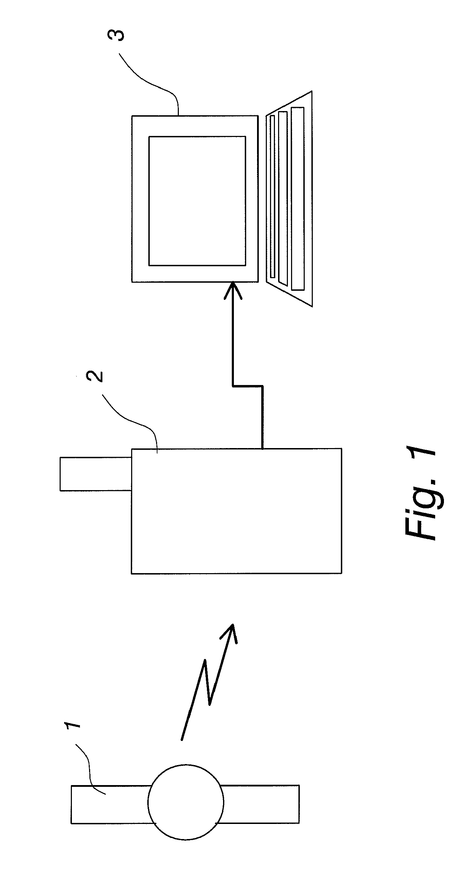

The invention will now be described in more detail with reference to the accompanying drawing, in which
- fig. 1: shows in a general block diagram some apparatus units used for implementing a method of the invention, and
- fig. 2: shows an algorithm for use in a data analysis according to the invention, whereby the measurement data is converted into a useful mode matching an objective of the invention.

In the described exemplary embodiment, the measuring device for a person's activity level is a wrist-held wrist unit 1, which is provided with one or more activity level measuring sensors. Such a sensor can be either a motion sensor for measuring a force between the hand and the unit 1 or it can be an acceleration sensor. Naturally, there may be several sensors and combinations thereof. The measured motion activity data is processed so as to retrieve motion activity readings from a measuring signal by integrating the measured information. In practice, this processing can be effected with a remote monitoring terminal 3, or with a server from which the results of an analysis are retrieved by means of a browser for the terminal device 3 employed in remote surveillance. The measuring results are transmitted from the wrist unit 1 as an RF transmission in a wireless manner to a local base station 2, which is in wired or wireless data transfer communication with the remote monitoring terminal 3.

In addition to motion measurement, the wrist unit 1 includes a hand-holding identification, which measured e.g. by capacitive measurement. The capacitance is higher when the hand is close by. This piece of information is also transmitted by way of the local base station 2 to the remote monitoring terminal 3.

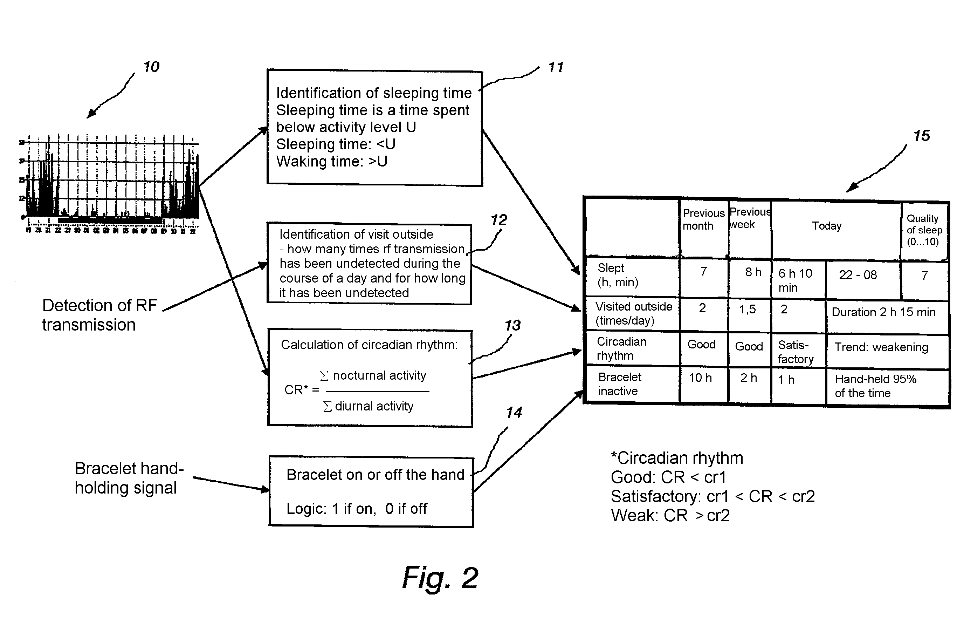

Fig. 2 shows a more detailed view of data processing algorithm blocks 11-14 used for analyzing a signal 10 transmitted from the wrist unit 1 by way of the base station 2.

In block 10 is performed a sleeping time identification, the sleeping time being interpreted as a time spent below a certain threshold level U. The waking time is a time spent above this threshold level U. In block 13 is performed a circadian rhythm calculation. The circadian rhythm is calculated by comparing nocturnal activity and diurnal activity. The circadian rhythm is good when diurnal activity exceeds nocturnal activity. The circadian rhythm is satisfactory with a reference value in the range of 1-2. The circadian rhythm is poor with a reference value higher than 2, in which case the sleeping time is not sufficient and the daytime activity is weak.

Also identified are a person's outside visiting times from non-detection times of the wrist unit transmission during the course of a day and from how long a non-detection period has been. The wrist unit sends a transmission at fixed times, yet in sufficient frequency, typically 3 times a minute, in order to provide a sufficiently accurate outside visit identification.

Moreover, in block 14 is identified an on-hand time for the wrist unit 1. Logic 1 in the bracelet hand-holding signal stands for a hand-contact time, and logic 0 indicates that the wrist unit is not in contact with the hand.

The above-mentioned analyzed information is used for presenting on the remote monitoring device's 3 display a detailed report 15, which can be e.g. in tabular format, and in which the analyzed information can be expressed in words and numbers. This information includes sleeping times, outside visit times, an estimate of circadian rhythm e.g. over the previous month, over the previous week, and today. In addition, the table shows how long the bracelet has been inactive, i.e. what is the hand-holding percentage of the time.

Hence, the remote terminal 3 enables an almost real-time surveillance over the quality of a person's sleep and circadian rhythm and its progression trend. The information can naturally be presented in a form other than the exemplary table 15. What is essential is that the data from information transmitted by the wrist unit 1 is refined into such a mode that the ultimately obtained information supports and enhances homecare services in the best possible manner.

Motion intensity readings are obtained from a measuring signal in practice by summing up the signals of various axes, thereby visualizing changes in activity, i.e. by performing a measurement for the extent of a motion vector. During sleep, the monitoring is focused on changes of position, which more easily reveal small movements, i.e. there is measured the turning of a vector in the Earth's gravity field. Thus, a small movement provides a large signal.

What is essential in the invention is that from the measuring signal is identified features, on the basis of which can be concluded matters essential from the standpoint of well-being, such as sleeping times, sleep quality, outside visiting times, circadian rhythm, etc. This identified information can then be used for displaying a detailed mode of representation or a digest in the form of an appropriate report. If necessary, the information can also be compiled for statistics. Setting information analysis criteria, such as threshold values, provides an opportunity of making useful conclusions about a person's physical state: is awake, is outside, is inside, is active, etc.

Moreover, within the report can be incorporated the time spent by a nurse at a care location. This can be included either in the same report view or the management may have its own view, in which this is also included.

## Claims

1. A method for producing information in support of homecare service for a person, said method comprising
- measuring the activity level with one or more motion and/or acceleration sensors
- processing the measurement data for obtaining motion intensity readings from a measuring signal
- interpreting that the person is outside when the measuring signal is no longer detected or when the signal strength is below a given level,
**characterized in that** the measuring result is compared with one or more threshold values and, as a result of the comparison, conclusions are made regarding the physical state of a person, **in that** said conclusions include sleeping times, outside visiting times, and circadian rhythm which is calculated by comparing nocturnal activity and diurnal activity, and **in that** the results of conclusion are displayed on a remote monitoring terminal as a detailed report, e.g. in tabular format.

2. A Method according to claim 1, **characterized in that** an activity level measuring device (1) is held around the wrist and the result report includes also a piece of information about the wrist-holding time.

3. A method according to claim 1 or 2, **characterized in that** the activity level measuring device is held around the wrist and the measurement results are transmitted from the measuring device (1) to a local base station (2), which transmits the information to an analyzing device, and the analyzed information is presented on the remote monitoring terminal's display device (3).

4. A method according to claim 1 or 2, **characterized in that** the activity level measuring device (1) is held around the wrist and the measurement results are transmitted from the measuring device to a local base station (2), which transmits the information to a server, **in that** the information is analyzed in the server, from which the analysis results are retrieved by means of a browser connected with a remote display device for the results.
